# EUROPÄISCHE PATENTSCHRIFT

(11) **EP 0 956 357 B1**
(45) Veröffentlichungstag und Bekanntmachung des Hinweises auf die Patenterteilung: **02.11.2005**
(21) Anmeldenummer: 97935555.9
(22) Anmeldetag: 30.07.1997
(51) Int. Cl.: C12N 15/82, C07K 14/415, C12Q 1/68, C07K 16/16, C12N 1/21, C12N 9/26, A01H 5/00

(54) **INVERTASE-INHIBITOR**
INVERTASE INHIBITOR
INHIBITEUR D'INVERTASE

(30) Priorität: 30.07.1996 DE 19630738; 07.10.1996 DE 19641302
(43) Veröffentlichungstag der Anmeldung: 17.11.1999
(73) Patentinhaber: Universität Heidelberg, 69117 Heidelberg (DE)
(72) Erfinder: RAUSCH, Thomas, D-69115 Heidelberg (DE); KRAUSGRILL, Silke, D-60316 Frankfurt (DE); GREINER, Steffen, D-63071 Offenbach (DE)
(74) Vertreter: Müller-Boré & Partner Patentanwälte
(86) Internationale Anmeldenummer: PCT/EP1997/004153
(87) Internationale Veröffentlichungsnummer: WO 1998/004722

(56) Entgegenhaltungen:
- EP-A- 0 677 581
- WO-A-92/14831
- WO-A-93/06711
- WO-A-96/12812
- SANDER, A., ET AL . : "SUCROSE PROTECTS CELL WALL INVERTASE BUT NOT VACUOLAR INVERTASE AGAINST PROTEINACEOUS INHIBITORS" FEBS LETTERS, Bd. 385, 6.Mai 1996, Seiten 171-175, XP002049864
- WEIL, M., ET AL . : "A 17-kDa NICOTIANA TABACUM CELL-WALL PEPTIDE ACTS AS AN IN-VITRO INHIBITOR OF THE CELL-WALL ISOFORM OF ACID INVERTASE" PLANTA, Bd. 193, 1994, Seiten 438-445, XP002050466
- PRESSEY, R.: "INVERTASE INHIBITOR IN TOMATO FRUIT" PHYTOCHEMISTRY, Bd. 36, Nr. 3, 1994, Seiten 543-546, XP002050467
- NEWMAN, T., ET AL . : "GENES GALORE: A SUMMARY OF METHODS FOR ACCESSING RESULTS FROM LARGE-SCALE PARTIAL SEQUENCING OF ANONYMOUS ARABIDOPSIS cDNA CLONES" EMBL SEQUENCE DATA LIBRARY, 28.August 1995, HEIDELBERG, GERMANY, XP002050468
- KRAUSGRILL, S., ET AL . : "REGULATION OF CELL WALL INVERTASE BY A PROTEINACEOUS INHIBITOR" JOURNAL OF EXPERIMENTAL BOTANY, Bd. 47, August 1996, Seiten 1193-1198, XP002050469

## Beschreibung

Die vorliegende Erfindung betrifft eine Nukleinsäure, die mindestens eine für ein Polypeptid kodierende Nukleinsäuresequenz enthält, wobei das Polypeptid zur Reduzierung der enzymatischen Aktivität einer invertase befähigt ist, das Polypeptid selbst sowie transgene Pflanzen, die diese Nukleinsäuresequenz enthalten. Ferner betrifft die vorliegende Erfindung Verfahren zur Herstellung von solchen transgenen Pflanzen mit reduziertem, lagerungsbedingtem Saccharose-Verlust.

Während der Lagerung von Zuckerrüben (*Beta vulgaris*), im Zeitraum zwischen Ernte und Verarbeitung, kommt es durch Atmung bzw. Saccharose-Metabolismus zu einem Saccharose-Verlust von etwa 0,02% pro Tag. Mit diesem Verlust geht ferner eine signifikante Qualitätsminderung infolge der Zunahme reduzierender Zucker, insbesondere Fructose und Glucose, einher (Burba, M. (1976) Atmung und Saccharosestoffwechsel lagernder Zuckerrüben. Zeitschrift für die Zuckerindustrie 26: 647-658). Der erste metabolische Schritt beim Saccharose-Abbau während der Rübenlagerung ist die enzymatische Hydrolyse durch eine vakuoläre Invertase. Dieses Enzym wird in Rübengewebe nach Verwundung de novo synthetisiert (Milling, R.J., Leigh, R.A., Hall, J.L. (1993) Synthesis of a vacuolar acid invertase in washed discs of storage root tissue of red beet (*Beta vulgaris* L.). J. Exp. Bot. 44: 1687-1694). Da die Hauptmasse der Rüben-Saccharose in den Zellvakuolen lokalisiert ist, spielt die (wund-)induzierte vakuoläre Invertase eine zentrale Rolle für den lagerungsbedingten Saccharose-Verlust.

Es gibt zur Zeit keine befriedigende Lösung für das Problem lagerungsbedingter Saccharose-Verluste (Burba, 1976). Die wichtigsten Maßnahmen im Stand der Technik bestehen in der Einhaltung niedriger Temperaturen (unter 12°C) und definierter Luftfeuchte (zwischen 90 und 96%). Jedoch sind alle bisher eingesetzten Maßnahmen zur Verminderung der lagerungsbedingten Verluste unbefriedigend.

Ein lagerungsbedingter Umsatz von Saccharose zu den Hexosen Glucose und Fructose und somit ein Saccharose-Verlust findet auch während des sogenannten "cold sweetening" bei Kartoffeln statt. Infolge der Kältebehandlung wird in den Kartoffelknollen eine vakuoläre Invertase induziert, die das Verhältnis von Saccharose zu Hexosen bestimmt (Zrenner, R., Schüler, K., Sonnewald, U. (1996) Soluble acid invertase determines the hexose-to-sucrose ratio in coldstored potato tubers. Planta 198: 246-252). Die Bildung der Hexosen als Folge des "cold sweetening" führt zu Qualitätseinbußen bei der Herstellung von beispielsweise Pommes frites.

Tomatenfrüchte (*Lycopersicon esculentum* Mill.) weisen einen hohen Wassergehalt auf. Dieser ist teilweise durch die osmotisch wirksamen endogenen Zucker (Saccharose und Hexosen) bedingt. Eine Erniedrigung des Gesamtzuckergehaltes durch Hemmung der Invertase-vermittelten Saccharose-Hydrolyse führt zu kleineren wasserärmeren Früchten (Klann, E.M., Hall, B., Bennett, A.B. (1996) Antisense acid invertase (TIV1) gene alters soluble sugar composition and size in transgenic tomato fruit. Plant Physiology 112: 1321-1330). Eine Verringerung des Wassergehaltes der Tomatenfrüchte führt zu einer Einsparung von Energiekosten bei der Herstellung von Fruchtkonzentraten (z.B. Ketchup). Da die Reduktion der vakuolären Invertase-Aktivität über Invertase-antisense Expression auf Grund des Vorkommens verschiedener Isoformen nur unvollständig gelingt, könnte die transgene Einführung eines Invertaseinhibitors große Vorteile mit sich bringen, insbesondere wenn dieser verschiedene Isoformen gleichermaßen hemmt.

Somit liegt der vorliegenden Erfindung die Aufgabe zugrunde, ein neues System bereitzustellen, das im wesentlichen keine lagerungsbedingten Saccharose-Verluste in Pflanzen hervorruft.

Diese Aufgabe wird durch die in den Patentansprüchen gekennzeichneten Gegenstände der vorliegenden Erfindung gelöst.

Ein erster erfindungsgemäßer Gegenstand betrifft eine Nukleinsäure, die mindestens eine für ein Polypeptid kodierende Nukleinsäuresequenz enthält, wobei das Polypeptid zur Reduzierung bzw. Erniedrigung der enzymatischen Aktivität einer Invertase befähigt ist.

Die Begriffe "Nukleinsäure" und "Nukleinsäuresequenz" bedeuten natürliche oder halbsynthetische oder synthetische oder modifizierte Nukleinsäuremoleküle aus Desoxyribonukleotiden und/oder Ribonukleotiden und/oder modifizierten Nukleotiden.

Der Begriff "Polypeptid" umfaßt natürlich vorkommende Polypeptide und rekombinante Polypeptide. Rekombinante Polypeptide bezeichnen ein mit molekularbiologischen Techniken hergestelltes Konstrukt, dem die natürliche DNA des originalen Genoms bzw. die natürliche DNA, modifiziert mit einer fremden DNA-Sequenz, zugrundeliegt, und rekombiniert werden kann, z.B. mit Plasmiden, und in einem geeigneten Wirtssystem repliziert und exprimiert werden kann.

Der Ausdruck "ein zur Reduzierung der enzymatischen Aktivität einer Invertase befähigtes Polypeptid" bedeutet ein Polypeptid, welches in Folge der Bindung an eine Invertase deren enzymatische Aktivität reduziert, wobei bei ausreichender Menge des Inhibitorproteins eine vollständige Inhibition möglich ist. Vorzugsweise soll durch die Inhibitorexpression in der transgenen Pflanze eine etwa 90%ige Inhibition der vakuolären Invertase erreicht werden.

In einer Ausführungsform der vorliegenden Erfindung ist die Invertase in einer Pflanzenzelle vakuolär lokalisiert. In einer anderen Ausführungsform ist die Invertase in der Zellwand lokalisiert. In einer weiteren Ausführungsform ist die Invertase im Cytosol lokalisiert. Die Invertase stammt vorzugsweise aus der Zuckerrübe, der Kartoffel oder der Tomate.

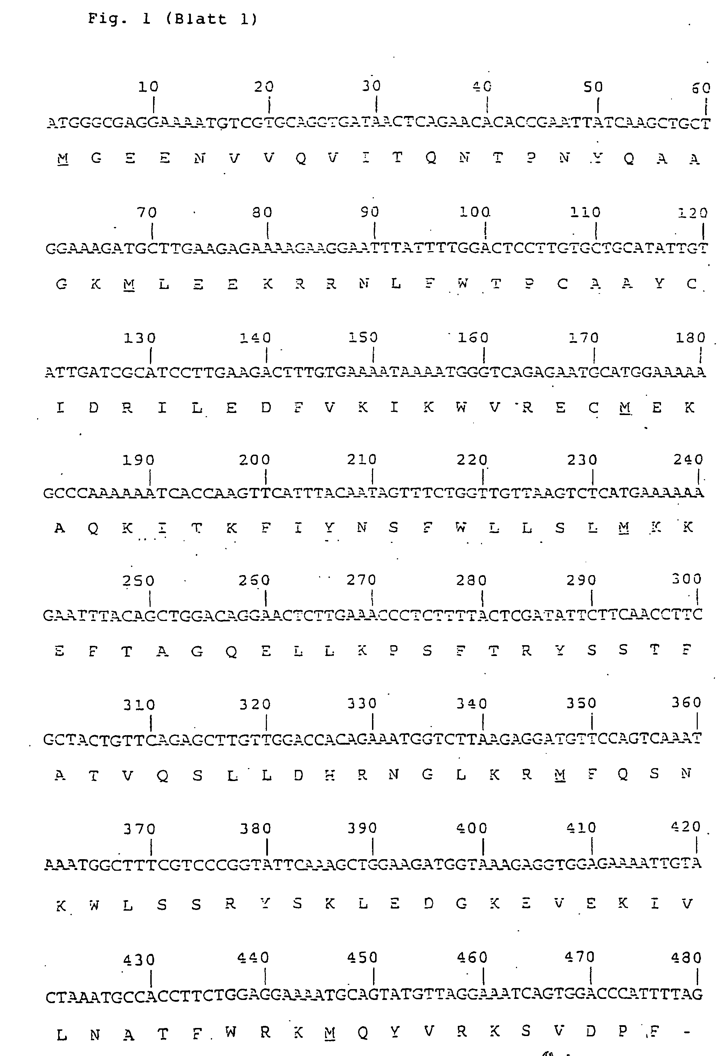

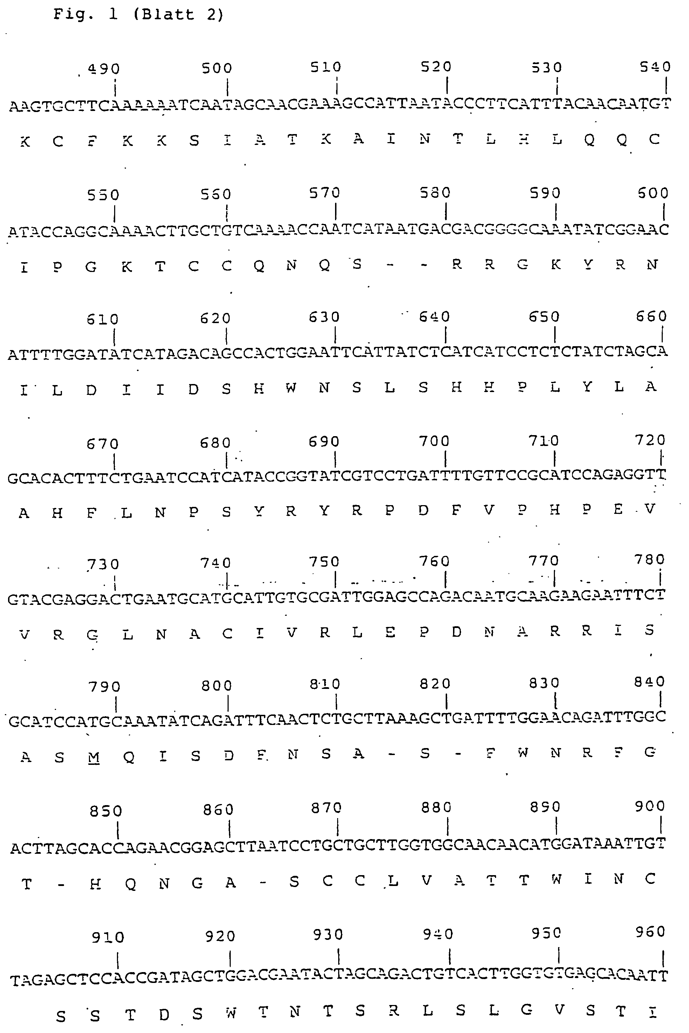

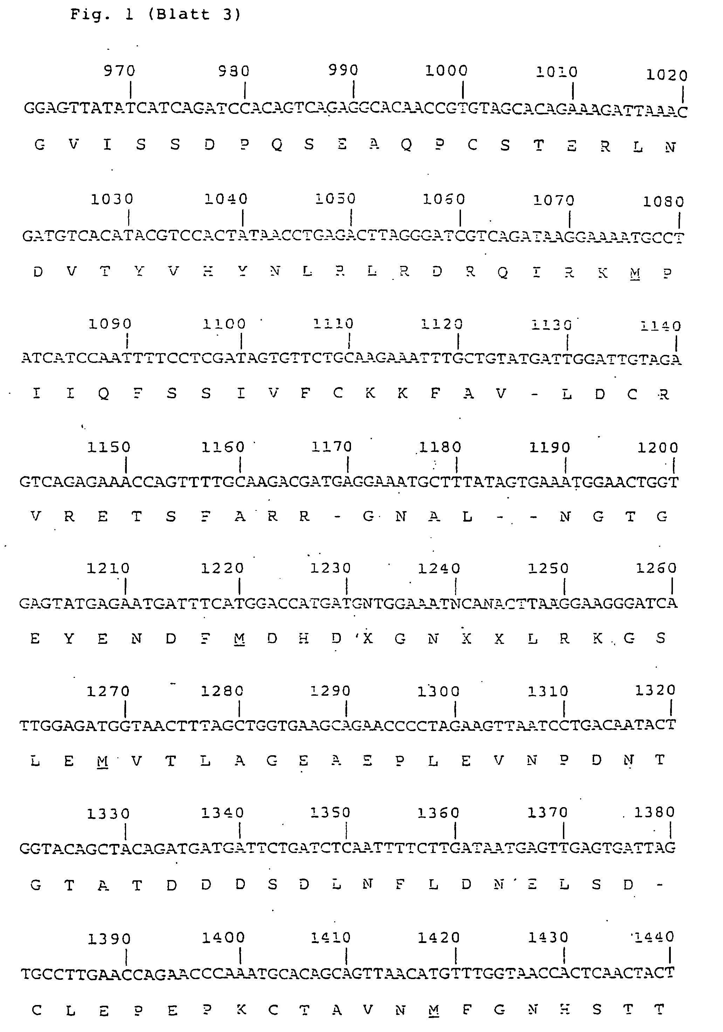

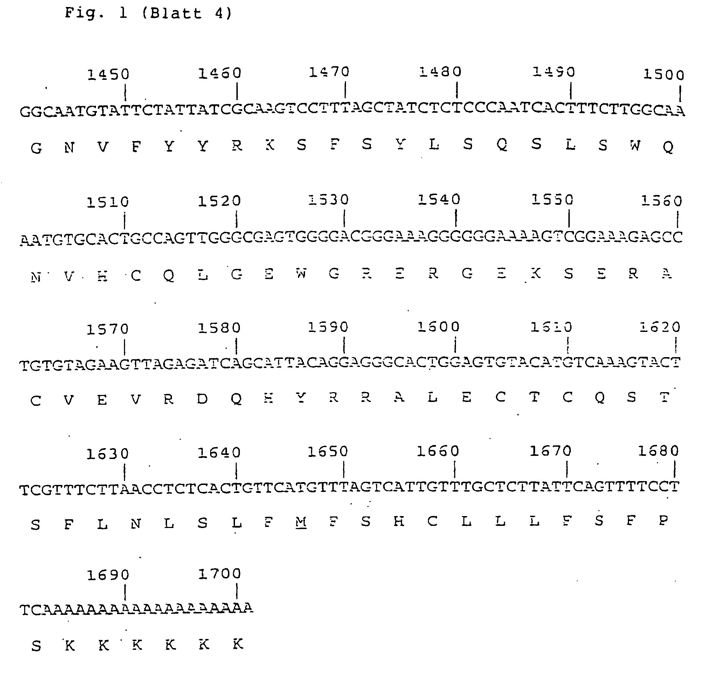

In einer bevorzugten Ausführungsform der vorliegenden Erfindung umfaßt die Nukleinsäure die in Figuren 1 (SEQ ID Nr. 1), 3 (SEQ ID Nr. 2), 12 (SEQ ID Nr. 3) und 14 (SEO ID Nr. 4) gezeigte Nukleinsäuresequenzen oder Abschnitte bzw. Fragmente davon sowie Nukleinsäuresequenzen, die mit den komplementären Sequenzen der in Fig. 1, 3, 12 oder 14 gezeigten Nukleinsäuresequenzen oder Abschnitten bzw. Fragmenten davon hybridisieren können.

In einer anderen Ausführungsform enthält die erfindungsgemäße Nukleinsäure eine weitere, für eine Targeting-Sequenz kodierende Nukleinsäuresequenz. Der Begriff "Targeting-Sequenz" bedeutet eine Aminosäuresequenz, welche die zelluläre Zielsteuerung in ein definiertes zelluläres Kompartiment vermittelt, beispielsweise die Zielsteuerung in die Vakuole.

In einer bevorzugten Ausführungsform der vorliegenden Erfindung umfaßt die Targeting-Sequenz die vakuoläre Targeting-Sequenz des Gerstenlektins mit folgender Aminosäuresequenz:

LEGVFAEIAASNSTLVAE

In einer anderen Ausführungsform enthält die erfindungsgemäße Nukleinsäure eine weitere, für ein Signalpeptid kodierende Nukleinsäuresequenz. Der Begriff "Signalpeptid" bedeutet eine hydrophobe Aminosäuresequenz, die vom Signalerkennungspartikel (SRP) erkannt wird. Das SRP vermittelt die Synthese des Gesamtpolypeptids am rauhen endoplasmatischen Reticulum (ER), mit der Folge, daß das entstehende Polypeptid in das Lumen des ER entlassen wird.

In einer weiteren Ausführungsform enthält die erfindungsgemäße Nukleinsäure eine für eine ER-Retentionssequenz kodierende Nukleinsäuresequenz.

In einer bevorzugten Ausführungsform stammt das Signalpeptid von einer Invertase, vorzugsweise von der Zellwand-Invertase aus Tabak.

In einer anderen Ausführungsform der vorliegenden Erfindung enthält die Nukleinsäure eine weitere Nukleinsäuresequenz, welche einen zur Expression in Pflanzen geeigneten Promotor umfaßt. Dieser Promotor bzw. Promotorsequenz stammt vorzugsweise aus der gleichen Pflanze wie die Invertase. In einer besonders bevorzugten Ausführungsform der vorliegenden Erfindung ist der Promotor ein Kartoffel- oder Zuckerrüben-spezifischer Promotor.

Zusammenfassend kann die erfindungsgemäße Nukleinsäure die vorstehend definierte, das Polypeptid kodierende Nukleinsäuresequenz und gegebenenfalls die vorstehend definierte, eine Targeting-Sequenz kodierende Nukleinsäuresequenz und/oder die ein Signalpeptid kodierende Nukleinsäuresequenz und/oder den vorstehend definierten Promotor umfassen, wobei vorzugsweise alle eine Aminosäuresequenz kodierenden Nukleinsäuresequenzen im Leserahmen angeordnet sind und entsprechend dem genetischen Code degeneriert sein können.

Ein weiterer Gegenstand der vorliegenden Erfindung ist ein Vektor, der die vorstehend definierte, erfindungsgemäße Nukleinsäure zur Expression des rekombinanten Polypeptids in prokaryotischen oder eukaryotischen Wirtszellen enthält. Der erfindungsgemäße Vektor kann vorzugsweise geeignete regulatorische Elemente, wie Promotoren, Enhancer, Terminationssequenzen, enthalten. Der erfindungsgemäße Vektor kann beispielsweise ein Expressionsvektor oder ein Vektor zur vorzugsweise stabilen Integration der erfindungsgemäßen Nukleinsäure in das genetische Material einer Wirtszelle sein. Ein geeignetes Expressionssystem umfaßt beispielsweise das Ti-Plasmid oder ein binäres Plasmidsystem in *Agrobacterium tumefaciens* als Vektor zur stabilen Integration der erfindungsgemäßen Nukleinsäure in das genetische Material einer Pflanze. Weiterhin kann die erfindungsgemäße Nukleinsäure z.B. auch durch das Ri-Plasmid von *Agrobacterium rizogenes,* durch direkten Gentransfer mittels Polyethylenglykol, durch Elektroporation oder durch Partikelbeschuß in das genetische Material einer Pflanze eingeführt werden.

Ein weiterer Gegenstand der vorliegenden Erfindung ist eine Wirtszelle, welche die erfindungsgemäße Nukleinsäure oder den erfindungsgemäßen Vektor enthält. Geeignete Wirtszellen sind beispielsweise Prokaryonten, wie *E. coli*, oder eukaryotische Wirtszellen wie *Saccharomyces cerevisiae, Schizosaccharomyces pombe, Hansenula polymorpha, Pichia pastoris* und *Bacculovirus*-infizierte Insektenzellen.

Ein weiterer erfindungsgemäßer Gegenstand ist das Polypeptid selbst, das von der vorstehend definierten Nukleinsäuresequenz kodiert wird, wobei die Nukleinsäuresequenz entsprechend dem genetischen Code degeneriert sein kann. Das erfindungsgemäße Polypeptid enthält mindestens einen zur Reduzierung der enzymatischen Aktivität einer Invertase befähigten Aminosäure-Sequenzabschnitt. In einer besonders bevorzugten Ausführungsform umfaßt das Polypeptid die in Figuren 1 (SEQ ID Nr. 5), 3 (SEQ ID Nr. 6), 12 (SEQ ID Nr. 7) und 14 (SEQ ID Nr. 8) gezeigten Aminosäuresequenzen oder Abschnitte bzw. Fragmente davon. Ferner umfaßt der Begriff "Polypeptid" beispielsweise Isoformen aus der gleichen Pflanze sowie homologe Inhibitor-Sequenzen anderer Pflanzenarten, wobei die Homologie auf Proteinebene vorzugsweise > 70% ist.

In einer erfindungsgemäßen Ausführungsform enthält das Polypeptid weiter eine am C-Terminus des Polypeptids angeordnete Aminosäuresequenz, welche eine vorstehend definierte Targeting-Sequenz und/oder eine ER-Retentionssequenz, beispielsweise "KDEL", umfaßt, und/oder eine am N-Terminus des Polypeptids angeordnete Aminosäuresequenz, welche ein vorstehend definiertes Signalpeptid umfaßt.

Die erfindungsgemäße Nukleinsäuresequenz, der erfindungsgemäße Vektor sowie das erfindungsgemäße Polypeptid können durch im Stand der Technik bekannte Verfahren hergestellt werden.

Ein weiterer Gegenstand der vorliegenden Erfindung ist eine transgene Pflanze, die mindestens die vorstehend definierte, erfindungsgemäße Nukleinsäure enthält.

Der Begriff "transgene Pflanze" bzw. "Pflanze" umfaßt die ganze Pflanze als solche sowie deren Teile, wie Wurzel, Stengel, Blatt, organspezifisches Gewebe oder Zellen, deren vermehrungsfähiges Material, insbesondere Samen, und deren Keimlinge. Ferner umfaßt dieser Begriff Stärkeknollen und -wurzeln, beispielsweise Kartoffel, Batate und Maniok, und Zuckerpflanzen, beispielsweise Zuckerrohr und Zuckerrübe, sowie Tomate und Mais.

In einer bevorzugten Ausführungsform der vorliegenden Erfindung ist der Wildtyp der transgenen Pflanze eine Zuckerrübe, eine Tomate oder eine Kartoffel.

Ein weiterer Gegenstand der vorliegenden Erfindung betrifft ein Verfahren zur Herstellung der erfindungsgemäßen transgenen Pflanze, worin eine Pflanzenzelle durch stabile Integration der vorstehend definierten Nukleinsäure in das genetische Material transformiert wird und die transformierte Pflanzenzelle zur transgenen Pflanze regeneriert wird. Verfahren zur Herstellung transgener Pflanzen sind im Stand der Technik bekannt.

Ein weiterer Gegenstand der vorliegenden Erfindung betrifft die Verwendung der vorstehend definierten Nukleinsäure zur Herstellung einer transgenen Pflanze mit reduziertem lagerungsbedingtem Saccharose-Verlust.

Erfindungsgemäß kann festgestellt werden, daß die Verminderung lagerungsbedingter Saccharose-Verluste durch die Expression des vorstehend definierten Polypeptids als "Invertase-Inhibitorprotein" in transgenen Pflanzen überraschenderweise ein hochspezifisches, umweltschonendes Verfahren zur Verbesserung von beispielsweise der Zuckerrüben- bzw. Kartoffelknollenqualität darstellt. Für die Zuckerrübe wird durch die Effizienzsteigerung der Zuckergewinnung bei vorgegebener Ertragshöhe eine Verminderung des Produktionsmitteleinsatzes ermöglicht. Im Fall der Kartoffel wird durch Reduktion der Kälte-induzierten Hexose-Bildung die Produktqualität der Kartoffeln, insbesondere für die Herstellung von Pommes frites, erhöht. Im Fall der Tomate wird durch die Reduktion osmotisch wirksamer Hexosen der Wassergehalt der Tomatenfrucht erniedrigt.

Durch die Kombination der den Invertase-Inhibitor kodierenden Nukleinsäuresequenz mit einer, eine geeigneten Targeting-Sequenz kodierenden Nukleinsäuresequenz kann beispielsweise eine korrekte vakuoläre Zielsteuerung des exprimierten Invertase-Inhibitors in die Vakuole erreicht werden und somit die Expression des Invertase-Inhibitors räumlich begrenzt werden. Ferner kann durch Verwendung von beispielsweise rüben- oder knollenspezifischen Promotoren die Expression des Invertase-Inhibitors zeitlich begrenzt werden.

### Die Figuren zeigen:

Figur 1 zeigt die den Invertase-Inhibitor kodierende c-DNA aus *Nicotiana tabacum* mit einer Länge von 1701 bp, wobei der offene Leserahmen ("open reading frame", "ORF") 477 bp mit Startnukleotid 1 umfaßt. Der von dieser Nukleinsäuresequenz kodierte Invertase-Inhibitor weist 159 Aminosäuren mit einem errechneten Molekulargewicht M, von 18915 und einem errechneten isoelektrischen Punkt von 10,13 auf.
Figur 2 zeigt die schematische Herstellung des Inhibitor-Konstrukts einer bevorzugten erfindungsgemäßen Ausführungsform zur Transformation von Pflanzen.
Figur 3 zeigt eine weitere, für einen in der Zellwand von Tabakzellen lokalisierten Invertase-Inhibitor kodierende cDNA mit einer Länge von 631 bp (ohne poly-A). Die genutzte Signalsequenz für die Sekretion in die Zellwand ist fett markiert. Die Schnittstelle, welche mit dem ansequenzierten N-Terminus des reifen Proteins identisch ist, ist durch einen Pfeil markiert.
Figur 4 zeigt die Expression des rekombinanten Tabak-Invertaseinhibitors in *E.coli*. Die in Figur 3 dargestellte cDNA wurde in den pQE-Vektor (Qiagen, Hilden, Deutschland) kloniert. Das rekombinante Protein wurde als his-tagged Fusionsprotein exprimiert. A: M, Molekulargewichtsmarker; 1: Bakterien nichtinduziert; 2: Bakterien mit IPTG induziert; 3: Affinitätschromatographisch (Ni-NTA) gereinigter rekombinanter Tabak-Invertaseinhibitor. B: Western Blot-Analyse der Fraktionen 1-3 aus A mit einem gegen den Inhibitor gerichteten polyklonalen Antiserum.
Figur 5 zeigt die dosisabhängige Hemmung der Zellwand-Invertase aus Tabak durch das rekombinante Inhibitorprotein. Die Punkte zeigen die Hemmung nach Vorinkubation beider Proteine ohne Saccharose, die Vierecke zeigen die Hemmung ohne Vorinkubation.
Figur 6 zeigt die Induktion der sauren Invertaseaktivität in Zuckerrüben nach Verwundung.
Figur 7 zeigt die Hemmung der Gesamt-Invertaseaktivität aus verwundeten Zuckerrüben durch aus Tabakzellkulturen gewonnenem Invertaseinhibitor.
Figur 8 zeigt die Hemmung der Zellwand-Invertase aus der Zuckerrübe durch den rekombinanten Tabak-Invertaseinhibitor (siehe Figuren 3-5). Die Punkte zeigen die Hemmung nach Vorinkubation beider Proteine ohne Saccharose, die Vierecke zeigen die Hemmung ohne Vorinkubation.
Figur 9 zeigt die Hemmung der Gesamt-Invertaseaktivität aus verwundeten Zuckerrüben (vakuoläre Invertase + Zellwand-Invertase) durch den rekombinanten Tabak-Invertaseinhibitor (siehe Figuren 3-5). Die Punkte zeigen die Hemmung nach Vorinkubation beider Proteine ohne Saccharose, die Vierecke zeigen die Hemmung ohne Vorinkubation.
Figur 10 zeigt die immunologische Identifizierung der vakuolären Invertase (VI) aus Tomatenfrüchten, sowie den Nachweis eines zum Tabak-Invertaseinhibitor homologen Tomateninhibitors (INH). Beide Proteine wurden mit polyklonalen, monospezifischen Antiseren.detektiert. Die VI zeigt nach SDS-PAGE und Western Blot zwei Spaltprodukte von 52 und 20 KD. Die VI bindet vollständig an Concanavalin A-Sepharose, wohingegen der Tomaten-Invertaseinhibitor zu etwa gleichen Anteilen in der ConA-bindenden und der ConA-nichtbindenden Fraktion vorliegt.
Figur 11 zeigt die Hemmung der Tomaten-VI durch den rekombinanten Tabak-Invertaseinhibitor (siehe Figuren 3-5). Die Punkte zeigen die Hemmung nach Vorinkubation beider Proteine ohne Saccharose, die Vierecke zeigen die Hemmung ohne Vorinkubation.
Figur 12 zeigt die Sequenz einer partiellen cDNA für den Tomaten-Invertaseinhibitor, die über RT-PCR aus Tomatenblüten-cDNA amplifiziert wurde.
Figur 13 zeigt den Vergleich von zwei (identischen) partiellen Tomaten-Invertaseinhibitor-Klonen mit dem Tabak-Invertaseinhibitor (siehe Figur 3).
Figur 14 zeigt die cDNA Sequenz eines cytosolischen Homologs zum Invertaseinhibitor-Klon aus Fig. 3. Das durch diesen Klon kodierte Protein ist zur Hemmung cytosolischer Invertasen befähigt.

Durch das nachfolgende Beispiel wird die vorliegende Erfindung näher erläutert.

### Beispiel

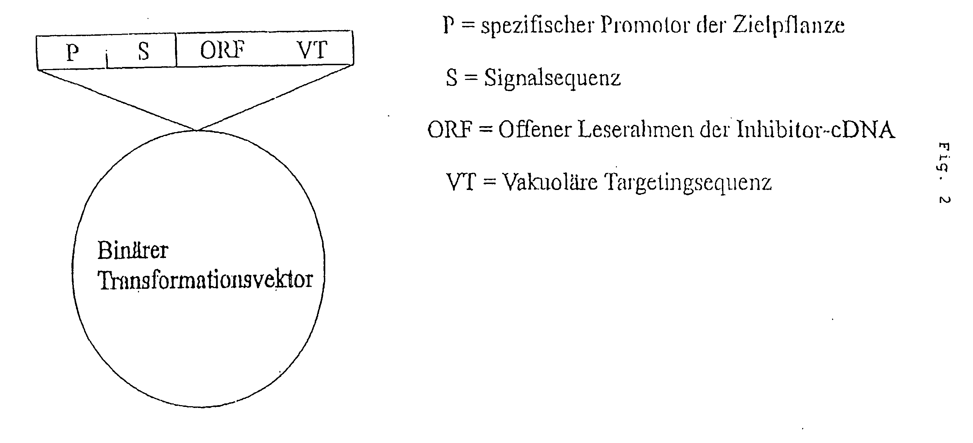

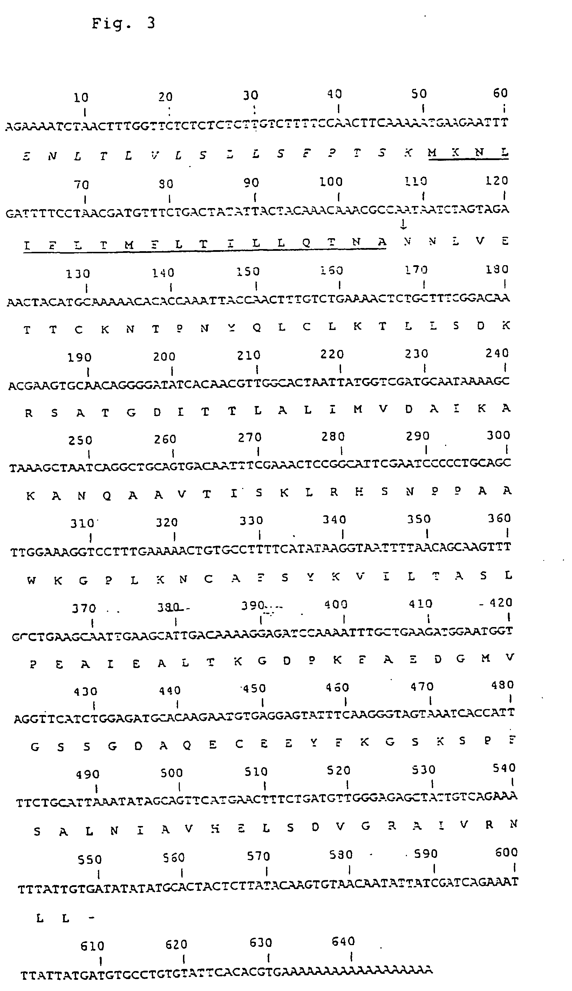

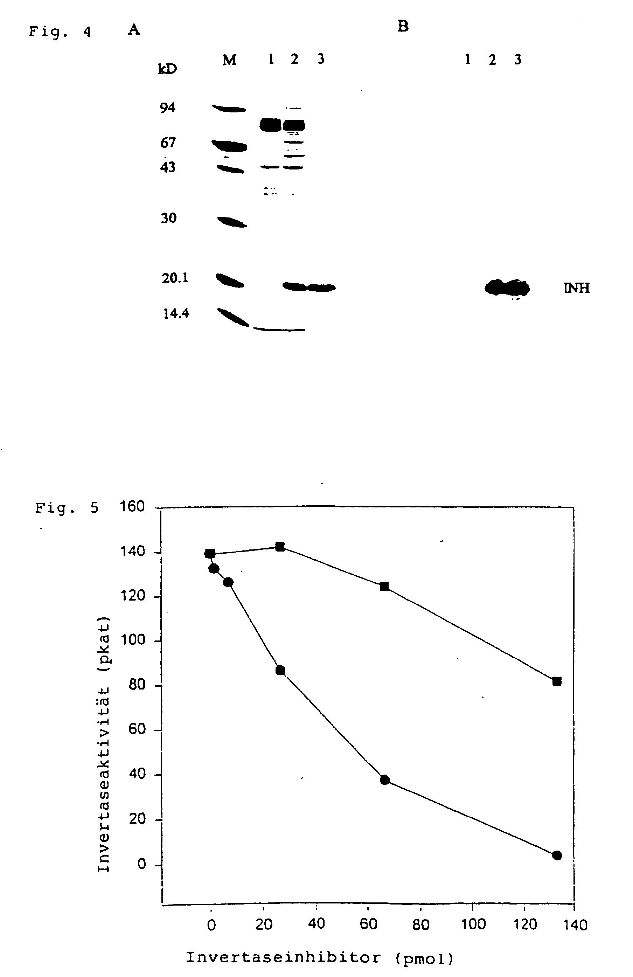

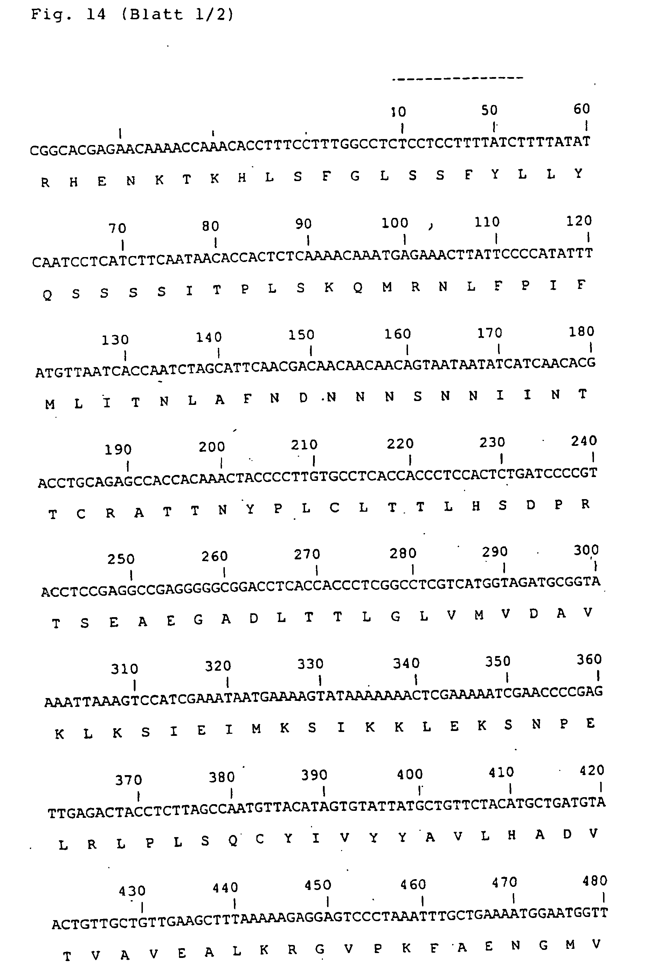

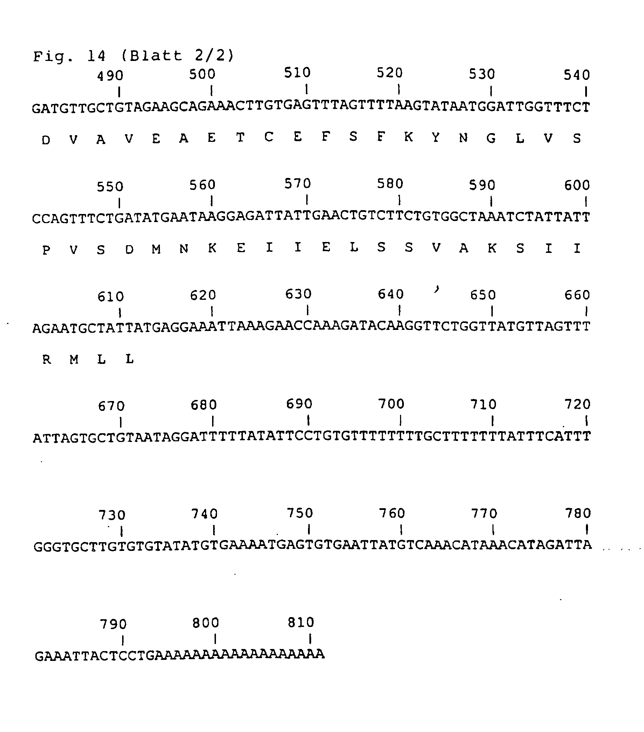

Sämtliche, im folgenden Beispiel zur Anwendung kommenden Methoden für die Herstellung der erforderlichen Genkonstrukte entsprechen Standardverfahren molekularbiologischen Arbeitens (Ausubel, F., Brent, R., Kingston, R.E., Moore, D.D., Seidmann, J.G., Smith, J.A., and Struhl, K. (1987-1996) *Current Protocols in Molecular Biology,* Greene Publishing). Der Arbeitsgang gliedert sich im wesentlichen in folgende Abschnitte:
(1) Das Inhibitorprotein wird über selektive Salzelution des Zellwandproteins, zweifache Ionenaustauscherchromatographie und anschließende SDS-Polyacrylamidgelelektrophorese bis zur Homogenität gereinigt.
(2) Das homogene Inhibitorprotein wird tryptisch verdaut und die erhaltenen Peptide des Inhibitorproteins werden über *Edman*-Abbau sequenziert.
(3) Ausgehend von den erhaltenen Peptidsequenzen werden degenerierte Primer synthetisiert und mit ihrer Hilfe über PCR DNA-Fragmente der Inhibitor-cDNA aus Gesamt-cDNA amplifiziert.
(4) Aus Tabakzellkulturen wird eine cDNA-Bank (in einem Expressionsvektor; ZAP Express®, Fa. Stratagene) hergestellt.
(5) Die erhaltenen Partialsequenzen der Inhibitor-cDNA (siehe (2)) werden für das Screening der cDNA-Bank eingesetzt.
(6) Der erhaltene Vollängenklon wird nach Expression in *E.coli* (Einklonierung in den pQE-Vektor der Fa. Qiagen) hinsichtlich seiner Funktion (Invertase-Inhibition) bestätigt.
(7) Der für das Inhibitorprotein kodierende Abschnitt des cDNA-Klons (Fig. 1) wird über PCR amplifiziert. Hierfür werden Primer mit Restriktionsschnittstellen eingesetzt, die die anschließende Ligation mit der Signal- und der Targeting-Sequenz erlauben. Am 5'-Ende wird mit der Signalsequenz, am 3'-Ende mit der Targeting-Sequenz für die Vakuole ligiert.
   *Gewinnung der Signalsequenz:* Die Signalsequenz wird mittels PCR aus der cDNA der Tabak-Zellwand-Invertase (Greiner, S., Weil, M., Krausgrill, S., Rausch, T. (1995) Plant Physiology 108: 825-826) amplifiziert (Bereich: Met¹-Val²³). Hierfür werden Primer mit Restriktionsschnittstellen eingesetzt, die die anschließende Ligation mit der Inhibitor cDNA erlauben. *Gewinnung der Targeting-Sequenz:* Die Targeting-Sequenz wird aus der cDNA für das Gerstenlektin amplifiziert (Bednarek, S.Y., Taikhel, N.V. (1991) Plant Cell 3: 1195-1206). Hierfür werden ebenfalls Primer mit Restriktionsschnittstellen eingesetzt, die die anschließende Ligation mit der Inhibitor cDNA erlauben.
   Zur *sense*-Klonierung der Nukleinsäure aus Fig. 3 (SEQ ID Nr. 2) wird die gesamte Nukleinsäure mit Hilfe der Restriktionsendonukleasen BamH I und Xba I aus dem pBK-CMV-Vektor (dieser generiert sich nach "in vivo excision" aus dem ZAP Express Phagen (Fa. Stratagene)) herausgeschnitten. Das erhaltene DNA-Fragment wird nun in einen BamH I/Xba I geschnittenen binären Transformationsvektor ligiert und anschließend in Bakterien transformiert.
   Zur *antisense*-Klonierung der Nukleinsäure aus Fig. 3 (SEQ ID Nr. 2) werden die Restruktionsendonukleasen BamH I und Kpn I verwendet. Ansonsten ist die Vorgehensweise die gleiche wie bei der *sense*-Klonierung.
   Die *sense-* und antisense-Klonierung der Nukleinsäure aus Fig. 14 (SEQ ID Nr. 4) erfolgt analog.
   Die so erhaltenen Konstrukte werden zum *Agrobacterium tumefaciens*vermittelten Gentransfer in-Pflanzen (im Beispiel Zuckerrübe, Kartoffel und Tomaten) eingesetzt.
   Die Einführung der vakuolären Targeting-Sequenz für die Nukleinsäuren aus Fig. 3 und 4 erfolgt wie für die Nukleinsäure aus Fig. 1 beschrieben.
(8) Das unter (7) genannte Genkonstrukt wird 5'-seitig mit einem rübenspezifischen Promotor ligiert und das erhaltene Konstrukt in einen binären Expressionsvektor einkloniert.
(9) Die Zielpflanze wird über eine geeignete, im Stand der Technik bekannte Transformationstechnik transformiert. Der Aufbau des für die Transformation benötigten Genkonstruktes ist in Fig. 2 dargestellt.

Gegen den in Tabakzellen exprimierten Invertaseinhibitor wurde ein Antiserum für das Screening einer cDNA-Bank hergestellt. Desweiteren wurden mit aus partiellen Aminosäuresequenzen abgeleiteten Oligonukleotiden PCR-Reaktionen zur Gewinnung einer homologen cDNA-Sonde durchgeführt. Außerdem wurde ein Oligo-Screening durchgeführt. Mit dem Oligo-Screening wurde der Klon in Fig. 1 isoliert. Über RT-PCR wurde ein 300 bp-Fragment amplifiziert, welches dann als Sonde für das Screening der cDNA-Bank eingesetzt wurde. Hierbei wurde der Klon in Fig. 3 isoliert. Letzterer wurde in *E.coli* als his-tagged Fusionsprotein exprimiert (Fig. 4). Das rekombinante Inhibitorprotein hemmt die Zellwand-Invertase aus Tabak, wobei für diese Invertase-Isoform ein partieller Substratschutz beobachtet wird (Fig. 5), der jedoch bei anderen vakuolären bzw. in der Zellwand lokalisierten Invertasen nicht auftritt (s.u.). Außerdem wurde ein cytosolisch lokalisiertes Homolog zu dem in Fig. 3 dargestellten Inhibitorklon isoliert (Fig. 14). Das durch diesen Klon kodierte Protein kann als Inhibitor für cytosolische Invertasen wirken.

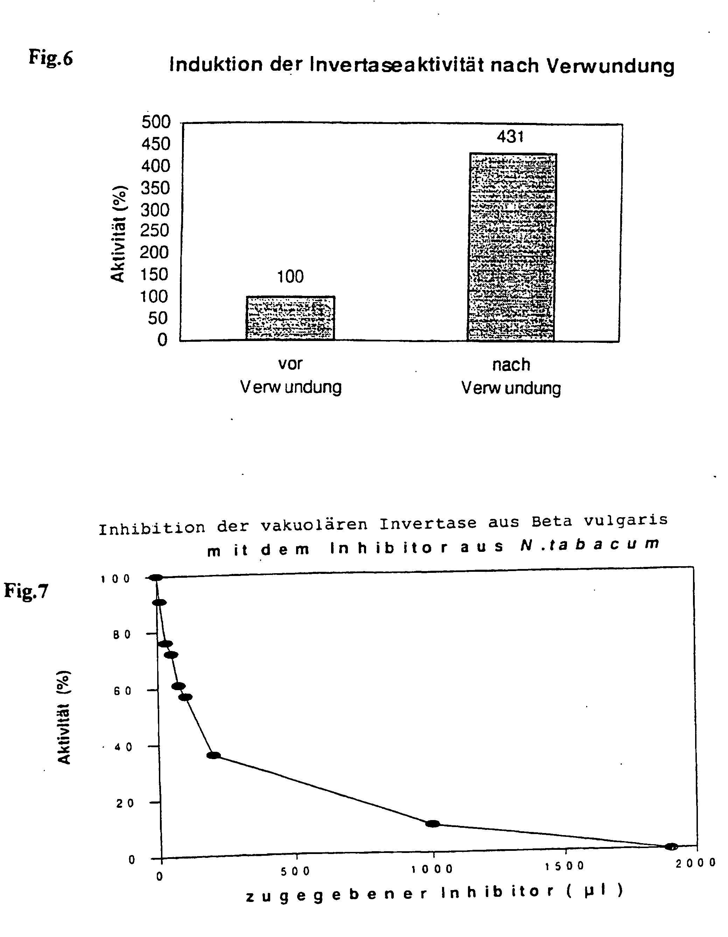

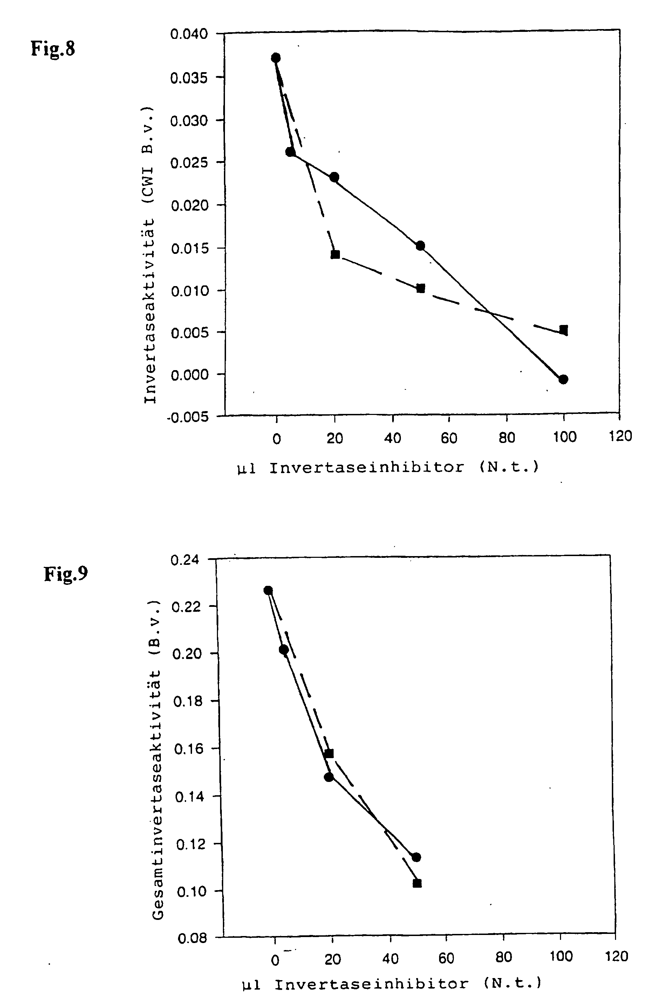

Die Invertase-Aktivität in verwundeten Zuckerrüben (Fig. 6) läßt sich durch das aus Tabakzellen isolierte (Fig. 7) Inhibitorprotein hemmen. Enzymkinetiken mit rekombinanten Tabak-Inhibitorprotein bestätigen, daß sowohl die Gesamt-Invertaseaktivität (Fig. 9) in verwundeten Zuckerrüben, wie auch die partiell gereinigte Zellwand-Invertase der Zuckerrübe (Fig. 8) durch den Invertaseinhibitor aus Tabak zu hemmen sind.

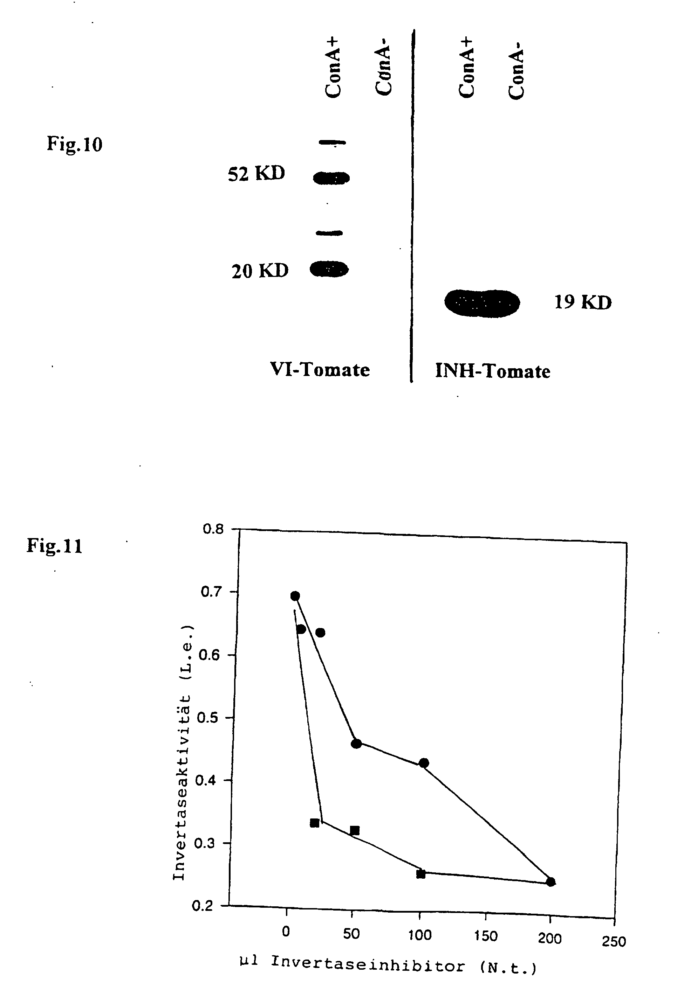

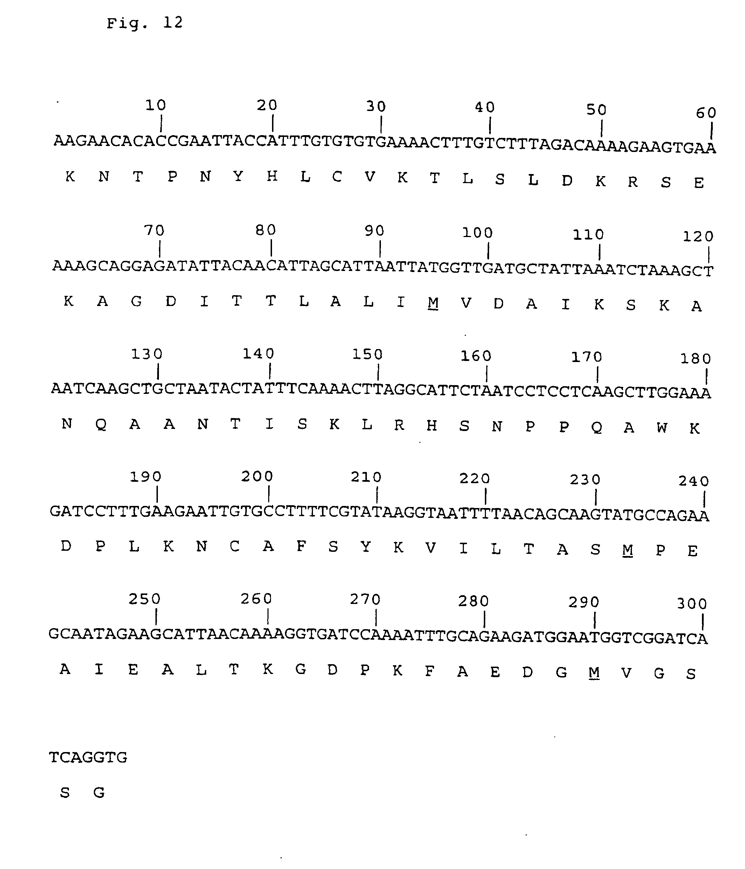

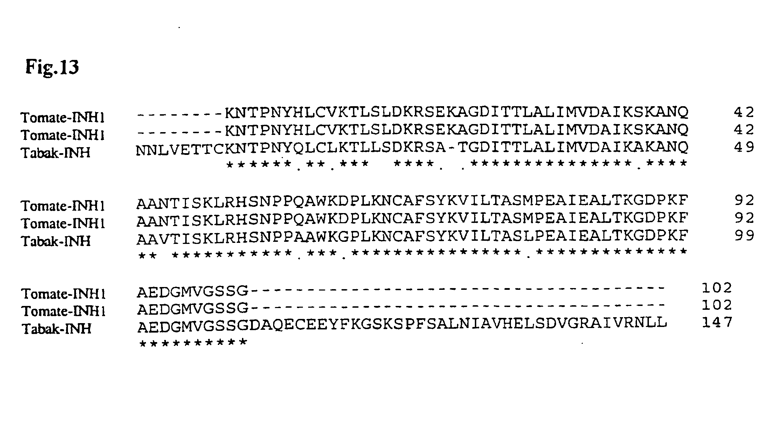

In Tomatenfrüchten wird in erster Linie vakuoläre Invertase exprimiert, die bei Auftrennung über SDS-PAGE in zwei Spaltprodukte zerfällt (52 und 20 KD; Fig. 10). Neben der vakuolären Invertase wird auch ein vermutlich im Zellwandraum lokalisierter Invertaseinhibitor von ca. 19 KD exprimiert, der mit dem Antiserum gegen den Tabak-Invertaseinhibitor kreuzreagiert (Fig. 10). Die aus Tomatenfrüchten isolierte vakuoläre Invertase wird ebenfalls durch den rekombinanten Tabak-Invertaseinhibitor gehemmt (Fig. 11). Die auffällige Sequenzhomologie einer über RT-PCR erhaltenen Tomaten cDNA-Partialsequenz mit der Sequenz des Tabak-Invertaseinhibitors (Fig. 12 und 13) stützt die Vermutung, daß der in Früchten exprimierte Tomaten-Invertaseinhibitor gegenüber der vakuolären Invertase kompartimentiert sein könnte (im Zellwandraum) und daher *in vivo* die vakuoläre Invertase nicht hemmt.

Zusammenfassend kann festgestellt werden, daß der apoplastische Tabak-Invertaseinhibitor (Fig. 3) nachgewiesenermaßen sowohl Zellwand-Invertasen wie auch vakuoläre Invertasen, insbesondere der Zuckerrübe und der Tomate, vollständig zu hemmen vermag. Das korrekte zelluläre Targeting vorausgesetzt, kann der Tabak-Inveftaseinhibitor in transgenen Pflanzen (Zuckerrübe, Kartoffel, Tomate) somit zur Verminderung der vakuolären und/oder in der Zellwand lokalisierten Invertasen eingesetzt werden. Der cytosolisch lokalisierte Invertaseinhibitor (Fig. 14) reguliert cytosolische Invertasen. Auch deren Hemmung in transgenen Pflanzen kann sich vorteilhaft auf das Saccharose/Hexose-Verhältnis auswirken.

## Patentansprüche

1. Verwendung einer Nukleinsäure, die für ein zur Reduzierung der enzymatischen Aktivität einer Invertase befähigtes Polypeptid kodiert, zur Herstellung einer transgenen Pflanze mit reduziertem, lagerungsbedingten Saccharose-Verlust.

2. Verwendung einer Nukleinsäure, die für ein zur Reduzierung der enzymatischen Aktivität einer Invertase befähigtes Polypeptid kodiert, zur Reduktion der Kälte-induzierten Hexose-Bildung in Kartoffeln.

3. Verwendung nach Anspruch 1 oder 2, wobei die Nukleinsäure mindestens die in Figur 1 (SEQ ID Nr. 1), 3 (SEQ ID Nr. 2), 12 (SEQ ID Nr. 3) oder 14 (SEQ ID Nr. 4) gezeigte, für ein Polypeptid kodierende Nukleinsäuresequenz oder Abschnitte davon enthält oder eine Nukleinsäuresequenz enthält, die mit den komplementären Sequenzen der in Figur 1 (SEQ ID Nr. 1), 3 (SEQ ID Nr. 2), 12 (SEQ ID Nr. 3) oder 14 (SEQ ID Nr. 4) gezeigten Nukleinsäuresequenzen oder Abschnitten davon hybridisieren kann, wobei das Polypeptid zur Reduzierung der enzymatischen Aktivität einer Invertase befähigt ist.

4. Verwendung nach Anspruch 3, wobei die Invertase in einer Pflanzenzelle vakuolär, im Cytosol oder Zellwand-lokalisiert ist.

5. Verwendung nach Anspruch 3 oder 4, wobei die Invertase aus der Zuckerrübe, der Kartoffel oder der Tomate stammt.

6. Verwendung nach einem der Ansprüche 3 bis 5, wobei die Nukleinsäure weiter mindestens eine für eine Targeting-Sequenz kodierende Nukleinsäuresequenz enthält.

7. Verwendung nach Anspruch 6, wobei die Targeting-Sequenz die folgende vakuoläre Targeting-Sequenz des Gerstenlektins umfaßt:

LEGVFAEIAASNSTLVAE

8. Verwendung nach einem der Ansprüche 3 bis 7, wobei die Nukleinsäure weiter mindestens eine für ein Signalpeptid kodierende Nukleinsäuresequenz enthält.

9. Verwendung nach Anspruch 8, wobei das Signalpeptid von einer Invertase stammt.

10. Verwendung nach Anspruch 8 oder 9, wobei das Signalpeptid von der Zellwand-Invertase aus Tabak stammt.

11. Verwendung nach einem der Ansprüche 3 bis 10, weiter enthaltend mindestens eine für eine ER-Retentionssequenz kodierende Nukleinsäuresequenz.

12. Verwendung nach einem der Ansprüche 3 bis 11, weiter enthaltend mindestens eine Nukleinsäuresequenz, welche einen zur Expression in Pflanzen geeigneten Promotor umfaßt.

13. Verwendung nach Anspruch 12, wobei der Promotor aus der gleichen Pflanze stammt wie die invertase.

14. Verwendung nach Anspruch 12 oder 13, wobei der Promotor ein Kartoffel-, Tomaten-, oder Zuckerrüben-spezifischer Promotor ist.

15. Verfahren zur Verminderung lagerungsbedingter Saccharose-Verluste, umfassend die Expression eines von einer Nukleinsäure kodierten Polypeptids in einer transgenen Pflanze, enthaltend mindestens die in das genetische Material stabil integrierte, nach einem der Ansprüche 1 bis 14 definierte Nukleinsäure, wobei das Polypeptid zur Reduzierung der enzymatischen Aktivität einer Invertase befähigt ist.

16. Verfahren zur Verbesserung der Zuckerrübenqualität, umfassend die Expression eines von einer Nukleinsäure kodierten Polypeptids in einer transgenen Pflanze, enthaltend mindestens die in das genetische Material stabil integrierte, nach einem der Ansprüche 1 bis 14 definierte Nukleinsäure, wobei das Polypeptid zur Reduzierung der enzymatischen Aktivität einer Invertase befähigt ist und wobei die transgene Pflanze eine Zuckerrübe ist.

17. Verfahren zur Verbesserung der Kartoffelknollenqualität, umfassend die Expression eines von einer Nukleinsäure kodierten Polypeptids in einer transgenen Pflanze, enthaltend mindestens die in das genetische Material stabil integrierte, nach einem der Ansprüche 1 bis 14 definierte Nukleinsäure, wobei das Polypeptid zur Reduzierung der enzymatischen Aktivität einer Invertase befähigt ist und wobei die transgene Pflanze eine Kartoffel ist.

## Claims

1. Use of a nucleic acid which codes for a polypeptide capable of reducing enzymatic activity of an invertase, for generating a transgenic plant with a reduced storage-related sucrose loss.

2. Use of a nucleic acid which codes for a polypeptide capable of reducing enzymatic activity of an invertase, for reducing the cold-induced hexose formation in potatoes.

3. Use according to Claim 1 or 2, where the nucleic acid comprises at least the nucleic acid sequence which codes for a polypeptide and which is shown in Figures 1 (SEQ ID No. 1), 3 (SEQ ID No. 2), 12 (SEQ ID No. 3) or 14 (SEQ ID No. 4) or segments thereof or comprises a nucleic acid sequence which is capable of hybridization with the complementary sequences of the nucleic acid sequences shown in Figures 1 (SEQ ID No. 1), 3 (SEQ ID No. 2), 12 (SEQ ID No. 3) or 14 (SEQ ID No. 4) or segments thereof, the polypeptide being capable of reducing the enzymatic activity of an invertase.

4. Use according to Claim 3, where the invertase is localized in the vacuole, in the cytosol or the cell wall of a plant cell.

5. Use according to Claim 3 or 4, where the invertase is derived from sugar beet, potato or tomato stock.

6. Use according to one of Claims 3 to 5, where the nucleic acid furthermore comprises at least one nucleic acid sequence which codes for a targeting sequence.

7. Use according to Claim 6, where the targeting sequence comprises the following vacuolar targeting sequence of barley lectin:

LEGVFAEIAASNSTLVAE

8. Use according to one of Claims 3 to 7, where the nucleic acid furthermore comprises at least one nucleic acid sequence which codes for a signal peptide.

9. Use according to Claim 8, where the signal peptide is derived from an invertase.

10. Use according to Claim 8 or 9, where the signal peptide is derived from the tobacco cell wall invertase.

11. Use according to one of Claims 3 to 10, furthermore comprising at least one nucleic acid sequence which codes for an ER retention sequence.

12. Use according to one of Claims 3 to 11, furthermore comprising at least one nucleic acid sequence which comprises a promoter which is suitable for expression in plants.

13. Use according to Claim 12, where the promoter is derived from the same plant as the invertase.

14. Use according to Claim 12 or 13, where the promoter is a potato-, tomato- or sugar beet-specific promoter.

15. Method for reducing storage-related sucrose losses, comprising the expression of a polypeptide, encoded by a nucleic acid, in a transgenic plant, comprising at least the nucleic acid defined as in one of Claims 1 to 14 stably integrated into the genetic material, the polypeptide being capable of reducing the enzymatic activity of an invertase.

16. Method for improving sugar beet quality, comprising the expression of a polypeptide, encoded by a nucleic acid, in a transgenic plant, comprising at least the nucleic acid defined as in one of Claims 1 to 14 stably integrated into the genetic material, the polypeptide being capable of reducing the enzymatic activity of an invertase and the transgenic plant being a sugar beet.

17. Method for improving potato tuber quality, comprising the expression of a polypeptide, encoded by a nucleic acid, in a transgenic plant, comprising at least the nucleic acid defined as in one of Claims 1 to 14 stably integrated into the genetic material, the polypeptide being capable of reducing the enzymatic activity of an invertase and the transgenic plant being a potato.

## Revendications

1. Utilisation d'un acide nucléique codant pour un polypeptide capable de réduire l'activité enzymatique d'une invertase, pour la production d'une plante transgénique, avec une perte réduite de saccharose due au stockage.

2. Utilisation d'un acide nucléique codant pour un polypeptide capable de réduire l'activité enzymatique d'une invertase, pour la réduction de la formation d'hexose induite par le froid dans des pommes de terre.

3. Utilisation selon la revendication 1 ou 2, dans laquelle l'acide nucléique contient au moins la séquence d'acide nucléique codant pour un polypeptide, présentée sur la figure 1 (SEQ ID N° : 1), sur la figure 3, (SEQ ID N° : 2), sur la figure 12 (SEQ ID N° : 3) ou sur la figure 14 (SEQ ID N° : 4) ou des parties de celle-ci, ou une séquence d'acide nucléique pouvant s'hybrider avec les séquences complémentaires des séquences d'acide nucléique présentées sur la figure 1 (SEQ ID N° : 1), sur la figure 3 (SEQ ID N° : 2), sur la figure 12 (SEQ ID N° : 3) ou sur la figure 14 (SEQ ID N° : 4) ou des parties de celles-ci, le polypeptide étant capable de réduire l'activité enzymatique d'une invertase.

4. Utilisation selon la revendication 3, dans laquelle l'invertase est localisée dans une cellule végétale au niveau vacuolaire, dans le cytosol ou dans la paroi cellulaire.

5. Utilisation selon la revendication 3 ou 4, dans laquelle l'invertase provient de betterave à sucre, de pomme de terre ou de tomate.

6. Utilisation selon l'une quelconque des revendications 3 à 5, dans laquelle l'acide nucléique contient en outre au moins une séquence d'acide nucléique codant pour une séquence de ciblage.

7. Utilisation selon la revendication 6, dans laquelle la séquence de ciblage comprend la séquence de ciblage vacuolaire suivante de la lectine d'orge :

LEGVFAEIAASNSTLVAE

8. Utilisation selon l'une quelconque des revendications 3 à 7, dans laquelle l'acide nucléique contient en outre au moins une séquence d'acide nucléique codant pour un peptide de signal.

9. Utilisation selon la revendication 8, dans laquelle le peptide de signal provient d'une invertase.

10. Utilisation selon la revendication 8 ou 9, dans laquelle le peptide de signal provient de l'invertase de la paroi cellulaire de tabac.

11. Utilisation selon l'une quelconque des revendications 3 à 10, contenant en outre au moins une séquence d'acide nucléique codant pour une séquence de rétention ER.

12. Utilisation selon l'une quelconque des revendications 3 à 11, contenant en outre au moins une séquence d'acide nucléique qui comprend un promoteur approprié pour l'expression dans des plantes.

13. Utilisation selon la revendication 12, dans laquelle le promoteur provient de la même plante que l'invertase.

14. Utilisation selon la revendication 12 ou 13, dans laquelle le promoteur est un promoteur spécifique de la pomme de terre, de la tomate ou de la betterave à sucre.

15. Utilisation pour la réduction des pertes de saccharose dues au stockage, comprenant l'expression d'un polypeptide codant pour un acide nucléique dans une plante transgénique, contenant au moins l'acide nucléique défini selon l'une quelconque des revendications 1 à 14, intégré de façon stable dans le matériau génétique, le polypeptide étant capable de réduire l'activité enzymatique d'une invertase.

16. Utilisation pour améliorer la qualité de la betterave à sucre, comprenant l'expression d'un polypeptide codant d'un acide nucléique dans une plante transgénique, contenant au moins l'acide nucléique défini selon l'une quelconque des revendications 1 à 14, intégré de façon stable dans le matériau génétique, le polypeptide étant capable de réduire l'activité enzymatique d'une invertase et la plante transgénique étant une betterave à sucre.

17. Utilisation pour améliorer la qualité de la tubercule de pomme de terre, comprenant l'expression d'un polypeptide codant d'un acide nucléique dans une plante transgénique, contenant au moins l'acide nucléique défini selon l'une quelconque des revendications 1 à 14, intégré de façon stable dans le matériau génétique, le polypeptide étant capable de réduire l'activité enzymatique d'une invertase et la plante transgénique étant une pomme de terre.
